# EUROPEAN PATENT APPLICATION

(11) **EP 1 063 290 A1**
(43) Date of publication of application: **27.12.2000**
(21) Application number: 99939147.7
(22) Date of filing: 11.03.1999
(51) Int. Cl.: C12N 9/02, C12N 15/53, C07K 16/40, A61K 38/44

(54) **NOVEL DICARBONYLREDUCTASE AND GENE ENCODING THE SAME**

(30) Priority: 12.03.1998 JP 8019598; 03.12.1998 JP 36003298
(71) Applicant: TAISHO PHARMACEUTICAL CO., LTD, Tokyo 170-8633 (JP)
(72) Inventor: NAKAGAWA, Junichi, Taisho Pharmaceutical Co., Ltd., Tokyo 170-8633 (JP); YOSHIMOTO, Makoto, Taisho Pharmaceutical Co., Ltd., Tokyo 170-8633 (JP)
(74) Representative: HOFFMANN - EITLE
(86) International application number: JP9901194
(87) International publication number: WO9946369

(57) **Abstract**

The novel proteins of the invention are the first to be cloned from mammals that have dicarbonyl reductase activity, that have amino acid sequences showing approximately 70% homology with the known mouse lung-specific carbonyl reductase (CR), and that exhibit high reducing activity on diacetyl substrates. The proteins are also localized in the renal medullary papillary area, and their expression is renal-specific and significantly reduced in diabetes model mouse kidneys.

## Description

### Technical Field

The present invention relates to novel proteins with dicarbonyl reductase activity, and to genes coding therefor. The invention further relates to antibodies for the proteins and to antisense oligonucleotides for the genes. The invention still further relates to an AGE production detecting method using the genes, to dicarbonyl compound production inhibitors characterized by containing the proteins, to AGE production inhibitors, to AGE production regulators containing the antisense oligonucleotides, and to an AGE production regulator screening method. The invention even still further relates to a method for diagnosis of diabetes complications characterized by using the aforementioned genes, proteins and antibodies.

### Background Art

The reaction in which an amino acid reacts with a reducing sugar in a nonenzymatic manner to produce browning is generally known as the Maillard reaction. This reaction occurs ubiquitously in living bodies, and specifically it has been reported that when amino acids in a protein participate in a Maillard reaction, the reaction leads to a complex series of steps including dehydration, polymerization, etc., forming a characteristic yellowish brown substance. The substance is called an Advanced Glycation End-product (abbreviated to "AGE" throughout the present specification).

AGE is known to accumulate to high levels in diabetic patients, and because of its tendency to bind to hemoglobin and collagen, it is believed to be a cause of arteriosclerosis and the fibrosis and hardening seen with kidney failure, as complications of diabetes.

Dicarbonyl molecules have been suggested as important intermediates in such AGE production (glycation) (Kato, H., Hayase, F., et al.: 3-Deoxyglucosone and Intermediate Product of the Maillard Reaction. The Maillard Reaction in Aging, Diabetes and Nutrition. (Baynes J.W., et al. eds.), p58-83, Alan R. Liss, New York, 1989).

### Disclosure of the Invention

The present invention is the result of (1) the discovery that regulation and inhibition of the aforementioned AGE production is an effective means for inhibiting and treating kidney failure and arteriosclerosis occurring as complications of diabetes, and (2) the discovery of a substance that can regulate and inhibit AGE production; upon considering the possibility of developing a method for detection of diabetes complications due to AGE production, developing a diagnosis method and diagnostic agent for its symptoms and developing a treatment method and treatment agent for its symptoms, there have been provided new proteins with enzyme activity capable of regulating and inhibiting AGE production, as well as genes coding therefor.

In other words, as a result of diligent research upon such consideration, the present inventors have succeeded in isolating a gene expressed specifically in mouse kidneys, which is a gene (hereunder referred to as gene dcr1) coding for the new protein according to the invention (hereunder referred to as protein DCR1), and in determining the base sequence of the gene and the inferred amino acid sequence of protein DCR1 encoded by it. The present invention has been completed upon finding that this new protein DCR1 exhibits dicarbonyl reductase activity.

As a result of searching for a novel gene coding for a protein with the same function in human kidneys based on the base sequence data for gene dcr1, the inventors have further succeeded in isolating a novel gene (hereunder referred to as gene hdcr1) coding for a novel protein (hereunder referred to as protein hDCR1) exhibiting the same activity as the aforementioned protein DCR1, and in determining the inferred amino acid sequence of protein hDCR1 encoded by the gene.

According to the invention, the initially obtained mouse-derived protein DCR1 and human-derived hDCR1 have high amino acid sequence homology (approximately 85%), and both also have approximately 70% homology with the known mouse lung-specific carbonyl reductase (CR).

Moreover, proteins DCR1 and hDCR1 exhibit high reducing activity on diacetyl substrates, and therefore represent the first diacetyl reductase activity to be cloned from mammals.

Protein DCR1 is localized in the renal medullary papillary area (mouse tissue antibody staining method). Its expression is renal-specific (in Western blotting using various organ extracts other than kidneys), and its expression is significantly reduced in diabetes model mouse kidneys.

In other words, the present invention provides novel dicarbonyl reductases derived from mammalian animals including mice and humans (particularly providing diacetyl reductases having high reducing activity particularly for α-diketones with linear alkyl groups of 4-10 carbons, and having especially high reducing activity for aromatic orthoquinones. As such α -diketones there may be mentioned diacetyl, 2,3-pentanedione, 2,3-hexanedione, 3,4-hexanedione and 2,3-heptanedione; as aromatic orthoquinones there may be mentioned isatin, phenanthrenequinone, acenaphthenequinone, and the like), genes coding therefor, antibodies and antisense oligonucleotides therefor, dicarbonyl compound production inhibitors containing the dicarbonyl reductases as components, AGE production inhibitors, AGE production regulators, and a gene expression regulating factor screening method, gene expression regulating factors, a protein activity regulator screening method and protein activity regulators.

The invention further provides a gene coding for either of the proteins described in (a) and (b) below.
(a) A protein comprising the amino acid sequence set forth in SEQ ID NO: 2 in the Sequence Listing.
(b) A protein comprising an amino acid sequence which is the amino acid sequence set forth in SEQ ID NO: 2 in the Sequence Listing with a deletion, substitution or addition of one or more amino acids, and having dicarbonyl reductase activity.

The invention still further provides a gene that hybridizes with the aforementioned gene under stringent conditions and codes for a protein with dicarbonyl reductase activity.

The invention still further provides the proteins described in (a) and (b) below.
(a) A protein comprising the amino acid sequence set forth in SEQ ID NO: 2 in the Sequence Listing.
(b) A protein comprising an amino acid sequence which is the amino acid sequence set forth in SEQ ID NO: 2 in the Sequence Listing with a deletion, substitution or addition of one or more amino acids, and having dicarbonyl reductase activity.

The invention still further provides antibodies against the aforementioned protein.

The invention still further provides an antisense oligonucleotide having the full or partial sequence of the antisense chain for any of the aforementioned genes, which inhibits biosynthesis of the aforementioned protein.

The invention still further provides a dicarbonyl compound production inhibitor characterized by containing the aforementioned protein.

The invention still further provides an AGE production inhibitor characterized by containing the aforementioned protein.

The invention still further provides an AGE production regulator that contains the aforementioned antisense oligonucleotide.

The invention still further provides a gene expression regulating factor screening method that employs at least a portion of the aforementioned gene, as well as the gene expression regulating factor obtained by the method.

The invention still further provides a protein activity regulator screening method that employs at least a portion of the aforementioned protein, as well as the protein activity regulator obtained by the method.

Preferred modes of the present invention will now be explained in detail. Throughout this specification, genes that are DNA obtained by reverse transcription from naturally occurring mRNA (including DNA obtained by amplification of the DNA) will be referred to as cDNA when necessary for clarification.

The following abbreviations are used where necessary throughout this specification.

### Abbreviation

- DNA: deoxyribonucleic acid
- A: adenine
- C: cytosine
- G: guanine
- T: thymine
- Ala (A): alanine
- Arg (R): arginine
- Asn (N): asparagine
- Asp (D): aspartic acid
- Cys (C): cysteine
- Gln (Q): glutamine
- Glu (E): glutamic acid
- Gly (G): glycine
- His (H): histidine
- Ile (I): isoleucine
- Leu (L): leucine
- Lys (K): lysine
- Met (M): methionine
- Phe (F): phenylalanine
- Pro (P): proline
- Ser (S): serine
- Thr (T): threonine
- Trp (W): tryptophan
- Tyr (Y): tyrosine
- Val (V): valine

### Brief Description of the Drawings

Fig. 1 shows a comparison of the amino acid sequence of protein hDCR1 (human-derived, top) with the amino acid sequence of protein DCR1 (mouse-derived, bottom).
Fig. 2 shows the homology between the amino acid sequence of protein DCR1 and the amino acid sequence of lung-specific carbonyl reductase (CR) (mouse). The top row is protein DCR1 and the bottom row is carbonyl reductase. The underlined sections indicate the peptide sequences used for antibody preparation.
Fig. 3 shows the homology between the amino acid sequences at the substrate recognition sites of protein hDCR1 (human-derived, top), protein DCR1 (mouse-derived, middle) and mouse carbonyl reductase (CR, bottom). The arrows indicate the amino acid residues essential for hydrophobic interaction with the substrates.
Fig. 4 is an electrophoresis image showing the results of affinity purification of the recombinant protein DCR1.
Fig. 5 is an electrophoresis image showing the results of Western blot measurement of the expression of protein DCR1 in the major organs of healthy mice.
Fig. 6 is an electrophoresis image showing the results of Western blot measurement of the expression of protein DCR1 in kidney extracts from pathological model mice, in comparison to healthy mice.
Fig. 7 is a graph showing the pH dependency of dicarbonyl reductase activity of protein DCR1 using diacetyl as the substrate.

### Best Mode for Carrying Out the Invention

### (Cloning of gene dcr1)

Gene dcr1 according to the invention may be obtained by the following procedure.

That is, the gene (dcr1) may be isolated from a kidney-derived cDNA library as CDNA fragments containing the gene. The cDNA library used here by the present inventors was constructed from renal tissue around and including the renal glomeruli.

The method used to distinguish the cDNA of the cDNA library believed to include the gene specifically expressed in kidney tissue may be a method whereby the gene expression frequency is analyzed according to the method of Okubo et al. (Nature Genet., 2, 173(1992)). For example, (1) kidney-derived mRNA is used as a template for cDNA synthesis, using as the primer oligo dT linked to one end of a vector plasmid opened with an appropriate restriction enzyme, and the cDNA is then cut with restriction enzymes MboI and BamHI.

The vector is prepared using dam methylase-positive *E. coli* as the host, and therefore the A residue of the MboI recognition sequence "GATC" is methylated. MboI therefore cleaves only the newly synthesized cDNA portion. The vector has only one BamHI cleavage site near the end opposite from the end to which the oligo dT has been linked, and the enzyme consequently cleaves the vector at one location; if the BamHI recognition sequence is present in the newly synthesized cDNA portion, that site is also cleaved. BamHI and MboI both produce identical sticky ends each consisting of the sequence "GATC", and therefore after cleavage by both enzymes DNA ligase may be used to relink the plasmid.

(2) A cDNA library is constructed by transforming *E. coli* using the plasmid prepared in this manner.

The library therefore includes the region from the 3' polyA site of each mRNA to the site where the base sequence GATC first occurs at the 5' portion.

(3) A suitable number of recombinants are randomly selected from the obtained cDNA library, the cDNA in each recombinant is extracted and the full base sequence is determined.

The organ-specific genes and the high-expression genes are distinguished based on whether any of the cDNA fragments having the specific sequence determined in this manner are found among the randomly selected recombinants.

When this procedure is followed, it is then possible to extract the recombinant cDNA and determine the base sequence of the cDNA by any of various methods that are publicly known to those skilled in the art (the method described in Molecular Cloning, 2nd ed., Cold Spring Harbor Lab. Press, 1989, or other methods described in the technical literature containing standard protocols for those skilled in the art; these will hereunder be referred to as "common methods").

The total number of randomly selected recombinants in the method for distinguishing the high expression gene is suitably from a few hundred to about a thousand, but if necessary the number of recombinants treated may be even greater.

Specifically, as will be illustrated in the examples that follow, all of the base sequences of the cDNA fragments from a suitable number of recombinants were determined, and among them, the cDNA fragments having above a certain frequency of cDNA with the same sequence were selected out as candidate DNA fragments containing genes specifically expressed in the kidneys.

(4) As mentioned above, the cDNA fragments include a portion of the 3' end of the mRNA, and the base sequence data for that region (hereunder referred to as the 3' fragment) are used to obtain the full length cDNA, as explained below. That is, using the kidney cDNA library as the template, oligonucleotides of an appropriate length having sequences within the 3' fragment may be synthesized and used as primers in the PCR for amplification.

This can also be accomplished by using the 3' fragment as a probe for colony hybridization or plaque hybridization to the kidney cDNA library, and screening by a common method.

The cDNA fragment amplified by the aforementioned method may be incorporated into an appropriate vector (for example, pT7BlueT-vector, marketed by Novagene, Inc.) by a common method and the total base sequence thereof determined.

Here, by obtaining two independent clones of the recombinant DNA and determining the base sequences of each of the cDNA fragments, the sequence may be verified. The base sequence of the obtained gene dcr1 is set forth in SEQ ID NO: 1 in the Sequence Listing.

### (Cloning of gene hdcr1)

By using the base sequence data determined with mice (taking into consideration the codons used), it is possible to isolate a gene coding for dicarbonyl reductase with the same action even from a different species, by colony hybridization or Southern blot hybridization using a probe synthesized according to the amino acid sequence inferred from the base sequence, or by polymerase chain reaction (PCR) using a primer synthesized from the data.

That is, human kidney mRNA may be used for construction of a cDNA library, and then subcloning to an appropriate plasmid and determination of the base sequence, according to common methods.

The base sequence of the obtained gene hdcr1 is listed as sequence No.3 in the Sequence Listing.

The invention also encompasses DNA sequences including the obtained base sequences of dcr1 or hdcr1, DNA sequences that hybridize with those sequences or with fragments derived therefrom under standard conditions, and DNA sequences that do not hybridize with those DNA sequences under standard conditions because of degeneracy of the genetic code but that code for polypeptides with exactly the same amino acid sequence. Here, "under standard conditions" for hybridization means conditions commonly employed by those skilled in the art for detection of a specific hybridization signal, such as the conditions described by Sambrook et al. (Molecular Cloning, Cold Spring Harbor Laboratory Press, New York, USA, 2nd Ed., 1989), preferably the "stringent hybridization/non-stringent washing conditions" described by Sambrook et al. and well-known to those skilled in the art, and even more preferably "stringent hybridization/stringent washing conditions".

### (Detection of tissue distribution)

The full or partial base sequence of the obtained gene may be used to prepare a probe for detection of the tissue distribution of gene expression. For example, the probe may be labeled by common publicly known labeling means and the publicly known Northern blot method carried out. By using a specific standard substance it is possible to quantify the amount of gene expressed in specific tissues.

By thus detecting expression of the gene it is possible to confirm dicarbonyl reductase activity. When the activity is connected with regulation and inhibition of AGE production, detection and quantitation of the gene constitutes a method of detecting regulation and inhibition of AGE production.

Similarly, quantitation of the gene expression also constitutes a method of confirming the effects of AGE production regulators or inhibitors. In this case, it is possible to confirm whether or not an AGE production regulator or inhibitor has been administered, or whether any change has occurred in gene expression during a given period after administration.

Specifically, it is possible to confirm specific expression in kidney tissue of a gene believed to be present in a cDNA fragment selected by the method described above, by determining the frequency of kidney-specific expression of the cDNA sequence by Western hybridization. Here, a crude extract of each kidney is prepared and subjected to SDS-polyacrylamide gel electrophoresis (SDS-PAGE), and after transferring the protein on the gel to a membrane filter, an anti-peptide antibody is used as a probe for hybridization by a common method. The method described here is used in the manner illustrated in the examples to determine the kidney specificity of the cDNA sequence expression, thus allowing confirmation of specific expression in the kidney tissue.

### (Proteins DCR1 and hDCR1)

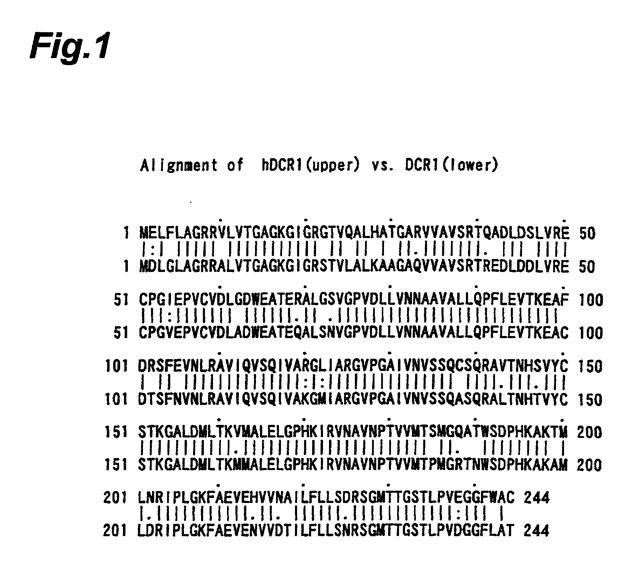

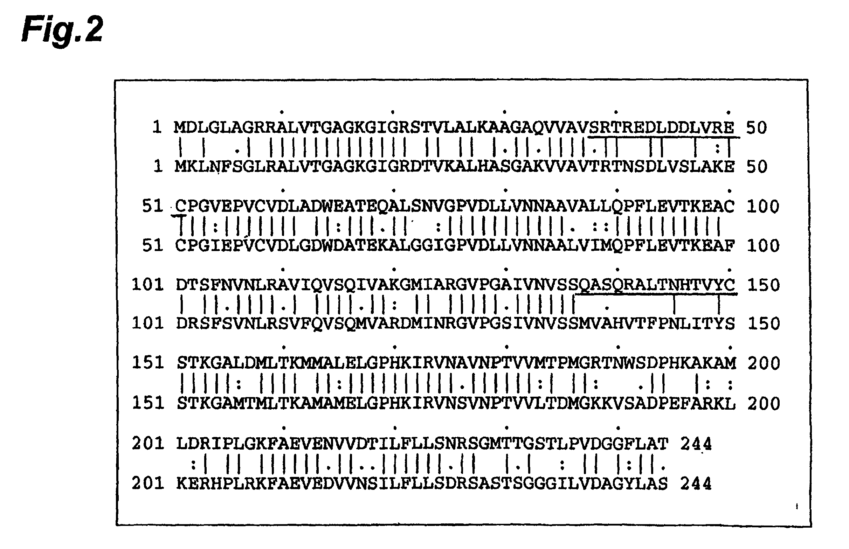

Genes dcr1 and hdcr1 are obtained in the manner described above, and the base sequence data for the genes are used to derive the amino acid sequences of the proteins coded for by the genes. Specifically, the proteins coded for by the new genes of the invention are proteins containing the amino acid sequences set forth in SEQ ID NOs: 2 and 4 in the Sequence Listing. The amino acid sequences of proteins DCR1 and hDCR1 exhibit high homology (Fig. 1). Fig. 2 shows the homology between the amino acid sequence of the obtained protein DCR1 and the amino acid sequence of mouse lung carbonyl reductase (CR). A high homology of approximately 70% was found. The underlined sections indicate the sequences used to prepare the anti-peptide antibodies explained below (#731 for the upper one and #733 for the lower one).

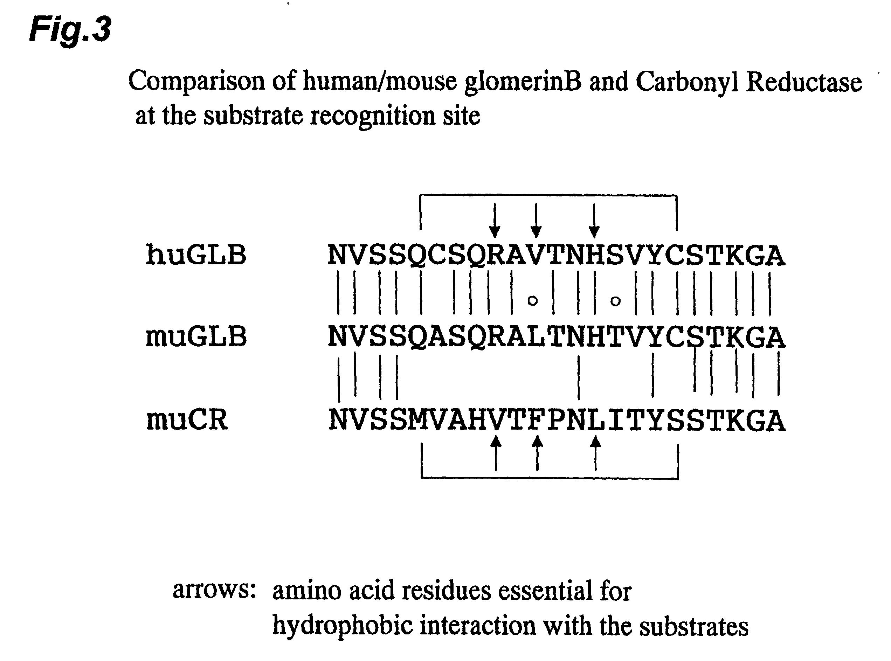

Fig. 3 shows a comparison of the amino acid sequences for the substrate recognition sites of mouse-derived DCR1, human-derived hDCR1 and mouse-derived carbonyl reductase.

The proteins of the invention are not limited only to the amino acid sequences set forth in SEQ ID NOs: 2 and 4 in the Sequence Listing, and include mutated proteins with a substitution, deletion or addition of one or more amino acids while still exhibiting dicarbonyl reductase activity.

Therefore, specific examples of genes (polynucleotides) coding for proteins with dicarbonyl reductase activity according to the invention include not only polynucleotides comprising the base sequence set forth in SEQ ID No: 1 in the Sequence Listing, but also mutated genes wherein a portion of the polynucleotide structure is altered by a natural or artificial mutation without altering the dicarbonyl reductase activity which is the major function of the polypeptide encoded by the polynucleotide.

An artificial mutation may be introduced by a common method.

Because of the degeneration of the genetic code, it is possible to obtain proteins with the same amino acid sequence from genes obtained by a substitution of different bases for at least some of the bases of the base sequence of the gene. Consequently, the genes coding for the proteins of the invention encompass all of the degenerate patterns that can code for the proteins and mutated proteins of the invention.

The method for obtaining the proteins (including mutated proteins) of the invention is not particularly restricted. Specifically there may be mentioned artificial peptide synthesis methods using protein synthesizers, or various protein expression methods involving genetic engineering techniques based on the base sequence data of the genes coding for the proteins, that are obtained according to the invention. In this case, the protein may be expressed either alone or as a fused protein such as MBP (Maltose Binding Protein), or it may be expressed with addition of a tag such as a FLAG peptide. A protein engineering method will allow preparation to be accomplished using a bacterium such as *E. coli* or a yeast, or animal cells or insect cells.

As a specific method there may be mentioned one in which an appropriate vector and host are selected and the gene is introduced to obtain transformants. (This can be accomplished, for example, by the protocol described in Molecular Cloning (Sambrook, et al., eds.) 1989, Cold Spring Harbor Laboratory Press, New York.) The obtained transformants may then be cultured, and the gene amplified and expressed for expression of the target protein.

The culture product may then be collected and, if necessary, concentrated, solubilized, dialyzed and subjected to various procedures such as chromatography to obtain the protein or mutated protein of the invention.

Several texts may be consulted for culturing of transformants, and for example, the protocol described in Molecular Cloning (Sambrook, et al., eds.) 1989, Cold Spring Harbor Laboratory Press, New York may be followed. The target protein (or mutated protein) based on the base sequence of the invention may also be expressed by a publicly known method.

For this procedure, the host used may be a bacterium such as *E. coli*, a yeast or animal cells, but animal cells are particularly preferred. The liposome method, electroporation or the like may be used for introduction of the gene into the cells. A method using DEAE-dextran (product of Pharmacia) is particularly preferred for use.

The purification method for purification of the protein from the obtained culture product may be immunoprecipitation, salting out, ultrafiltration, isoelectric point precipitation, gel filtration, electrophoresis, ion-exchange chromatography, or an affinity chromatography method such as hydrophobic chromatography or antibody chromatography, chromatofocusing, adsorption chromatography, reverse phase chromatography or the like, from among which appropriate selection may be easily made by a person skilled in the art.

During the production step, the resulting target protein may be produced by the transformant as a fused protein with another polypeptide. If necessary, it may be treated with a chemical substance such as bromcyan or with an enzyme such as a protease during the purification step to cut out the target protein.

### (Antibodies)

The method used to obtain antibodies for all or a part of the proteins of the invention (including the mutated proteins explained above) may be a common publicly known method. The antibodies of the invention include both polyclonal antibodies and monoclonal antibodies, so long as they react with the protein (or mutated protein) of the invention. They also include active fragments thereof and chimeric antibodies containing active fragments thereof.

Antibodies, or immunoglobulins, have H chains and L chains and are divided into 5 classes (IgA, IgD, IgE, IgG, IgM) based on their physicochemical and immunological properties. Of these, IgA and IgG are further divided into subclasses based on the type of their H chains. The new antibodies of the invention encompass all of these classes and subclasses.

The antibodies of the invention do not necessarily need to be used in the form of the full antibody molecules, and partial molecules (active fragments) may be used so long as they retain activity. As active fragments there may be specifically mentioned F(ab')2, Fab', Fab, Fv, recombinant Fv and single-stranded Fv. For example, digestion with pepsin gives F(ab)'2 and Fc', and digestion with papain gives Fab and Fc.

These active fragments may be used alone, but if necessary they may be bound to substances such as albumin or polyethylene glycol to be used in the form of new complexes. Such complexes usually exhibit maximum effects in the body because they remain for long periods without degradation. A method of adding substances such as albumin or polyethylene glycol to active fragments is described, for example, in "Antibodies, A Laboratory Manual" (Cold Spring Harbor Laboratory, 1988), p.77-81, p.129-137. Generally speaking, active fragments can be easily bound to albumin or the like by using a divalent reactive reagent such as SPDP (product of Pharmacia), SMPB (product of Pierce) or EMCS (product of Dotite).

The method of preparing the antibodies of the invention may be carried out with reference to, for example, "Antibody Experiment Protocols" (edited and published by the Japan Immunology Association). The immunogen may be a portion of the protein of the invention, i.e. a polypeptide comprising at least 8 contiguous amino acids of the amino acid sequence set forth in SEQ ID No: 2 in the Sequence Listing or of the amino acid sequence of a mutated form of the protein. The protein may be obtained by any method so long as it is of a purity suitable for preparation of antibodies.

When the immunogen is a polypeptide comprising from 8 to about 20 amino acids, this may be bound to a carrier such as Keyhole-Limpet Hemocyanin (KLH) for use as the antigen.

The animal immunized with the immunogen may be any one other than a human, and animal species capable of producing the target antibodies may be selected for use from among animals commonly used by those skilled in the art.

Polyclonal antibodies may be obtained by purifying the resulting antiserum. The purification may be accomplished by combining such methods as salting out, ion-exchange chromatography and affinity chromatography.

Monoclonal antibodies may be obtained by preparing fused cells by a common hybridoma preparation method and then inducing the cells to produce antibodies. The cell fusion may employ such means as polyethylene glycol, the Sendai virus, an electric pulse, etc.

The desired monoclonal antibodies can alternatively be obtained by using a genetic engineering method. For example, mRNA may be acquired from spleen cells or lymphocytes from an animal immunized with the protein of the invention or a portion thereof, or a hybridoma that produces the aforementioned monoclonal antibodies, and used to construct a cDNA library. Antibodies are then expressed by the cDNA library. Clones producing antibodies that react with the antigen are then screened out from the cDNA library, the obtained clones are cultured, and the target antibodies are purified from the culture mixture by a combination of such methods as salting out, ion-exchange chromatography and affinity chromatography.

When the starting material used is pathological tissue, the method employed may be, for example, *in situ* hybridization using the aforementioned probes. The ABC tissue staining method using the antibodies of the invention may also be employed.

### (Antisense oligonucleotide)

Antisense polynucleotides (or oligonucleotides) based on the genes obtained according to the invention encompass all those consisting of a plurality of nucleotides comprising bases, phosphates and sugars, including those not naturally occurring. Typically it refers to DNA and mRNA.

Antisense polynucleotide derivatives according to the invention encompass all those that are similar to polynucleotides in three-dimensional structure and function. This includes, for example, derivatives with other substances linked to the 3' end or 5' end of the polynucleotide, derivatives with non-naturally-occurring bases, sugars or phosphates, which include substances with substitution, deletion or addition modifications, for at least some of the bases, sugars or phosphates of the polynucleotide, and derivatives with backbones other than the sugar-phosphate backbone.

The antisense polynucleotides and their derivatives may be ones that hybridize to any portion of the gene or mutated gene of the invention.

The antisense polynucleotides and their derivatives may be used as investigative polynucleotide probes to determine the presence of genes coding for proteins of the invention or their mutated forms in tissue or cells, or the state of their expression.

They may also be used as diagnostic polynucleotide probes. Such probes preferably consist of at least 12 bases with a GC content of 30-70%, and more preferably consist of at least 16 bases with a GC content of 30-70%.

The antisense polynucleotides and their derivatives may also be used to regulate expression of proteins (or mutated proteins) of the invention. Since these would be expected to hybridize to genes and mRNA coding for the aforementioned proteins and thus inhibit expression of those proteins, they can be used as therapeutic agents for diseases associated with those proteins. That is, antisense drugs can be developed from these antisense polynucleotides and their derivatives.

The method employing a polynucleotide including a base sequence complementary to DNA or mRNA coding for a polypeptide to regulate expression of the polypeptide is generally referred to as the antisense method. The polynucleotide with the complementary sequence is bound to the DNA or mRNA carrying the genetic information during (1) the transcription stage from the gene to the pre-mRNA, (2) the processing stage from the pre-mRNA to the mature mRNA, (3) the nuclear membrane passing stage or (4) the translation stage to protein, thereby affecting the normal flow of genetic information transmission to regulate expression of the polypeptide.

Generally speaking, a base sequence consisting of 15 or more bases is considered to be a sequence with specificity. Thus, the antisense polynucleotides and antisense polynucleotide derivatives of the invention are assumed to bind specifically to mRNA for the genes of the invention so long as they include base sequences complementary to the mRNA for the proteins and mutated proteins of the invention and consisting of at least 15 bases.

On the other hand, if the length of the polynucleotide is too long it will be unsuitable for incorporation into the cells. While the antisense polynucleotides of the invention and their derivatives may be of any length, when it is considered that the antisense polynucleotides and antisense polynucleotide derivatives of the invention must be incorporated into cells to regulate expression of their target proteins, the antisense polynucleotides and antisense polynucleotide derivatives preferably consist of from 15 to 30 bases, more preferably from 15 to 25 bases and even more preferably from 18 to 22 bases that are complementary to mRNA for gene dcr1.

In addition to the antisense polynucleotides and antisense polynucleotide derivatives of the invention, publicly known antisense techniques may be employed to obtain various derivatives thereof designed to increase the effects of the polynucleotides as drugs, i.e. various derivatives with high binding strength to the target DNA or mRNA, tissue specificity, cell permeability, nuclease resistance and intracellular stability.

For easier hybridization, it is generally considered desirable to design polynucleotides or polynucleotide derivatives with base sequences that are complementary to the base sequences of stem-loop forming regions. The polynucleotides of the invention and their derivatives may also form stem-loops if necessary.

Polynucleotides with sequences complementary to sequences near translation initiation codons or at ribosome-binding sites, capping sites and splicing sites should provide high expression-inhibiting effects in most cases. Polynucleotides and polynucleotide derivatives of the invention that include sequences complementary to regions near translation initiation codons or at ribosome-binding sites, capping sites and splicing sites in genes coding for proteins or mutated proteins of the invention or in mRNA for those genes may be expected to exhibit high expression-inhibiting effects.

Current commonly known derivatives are preferred to be derivatives that provide an increase in at least the nuclease resistance, tissue selectivity, cell permeability or binding strength, and it is particularly preferred for the polynucleotide derivative to be a derivative having a phosphorothioate bond (see "Antisense -- From Technology to Therapy" (Schlingen and Siepen, et al., eds.) 1997, Blackwell Science, Berlin-Vienna) in the backbone structure. The polynucleotides of the invention and their derivatives also encompass derivatives with such functions and structures.

The method of producing an antisense polynucleotide derivative according to the invention may be the method described, for example, in "In Antisense Research and Applications" (Michael J. GAIT, p.290-299, CRC publications, Florida, 1993).

For wild-type DNA or RNA, an antisense polynucleotide of the invention may be obtained, for example, by synthesis using a chemical synthesizer or by PCR using as the template the gene coding for a protein of the invention. Some derivatives, such as methyl phosphonate-types or phosphothioate types, can be synthesized using a chemical synthesizer (for example, Model 394 by Perkin Elmer, Japan). In such cases, the target polynucleotide or polynucleotide derivative can be obtained according to the protocol described in the manual supplied with the chemical synthesizer, purifying the synthesized product by HPLC employing reverse phase chromatography or the like.

### (Dicarbonyl reductase activity)

The enzyme activity assay method is not particularly restricted, and it may be based on the activity assay method for mouse carbonyl reductase (CR) (Eur. J. Biochem. 22, 381-387(1995)). The enzyme used here was an expressed recombinant enzyme.
(a) Substrate specificity: In order to determine the substrate specificity, there may be used a hydrocarbon system including various ketone, aldehyde and ester groups, or an aromatic compound. According to the invention, the compounds listed in Table 1 below may be mentioned as preferred for use in determining substrate specificity. The table also shows results obtained for the substrate specificity of rat and human derived enzymes. Here, column "C" shows the substrate concentration (µM), column "RA" shows the relative activity (%) against a reference of 100 for diacetyl, and column "(Km)" shows the results of assay at pH 7.0, in mM.

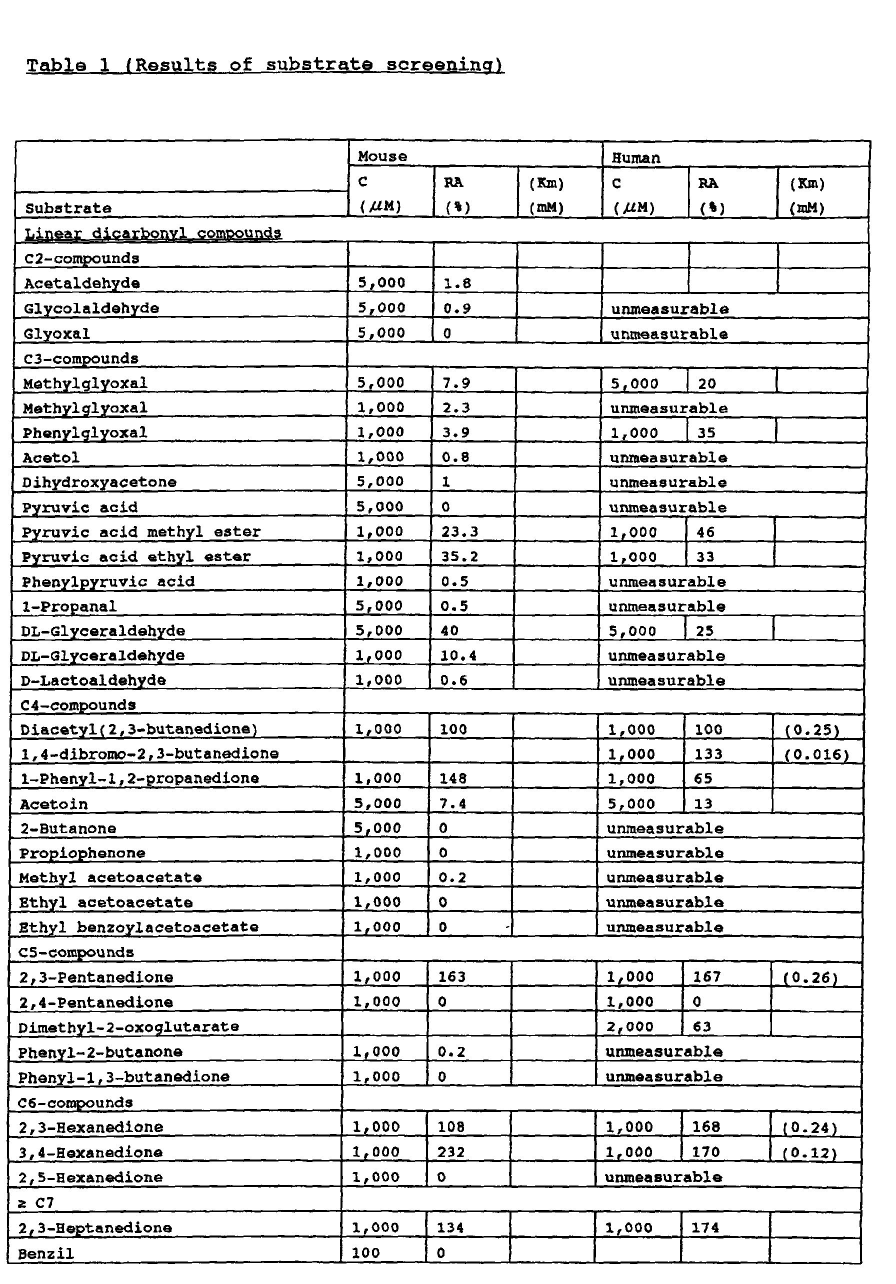

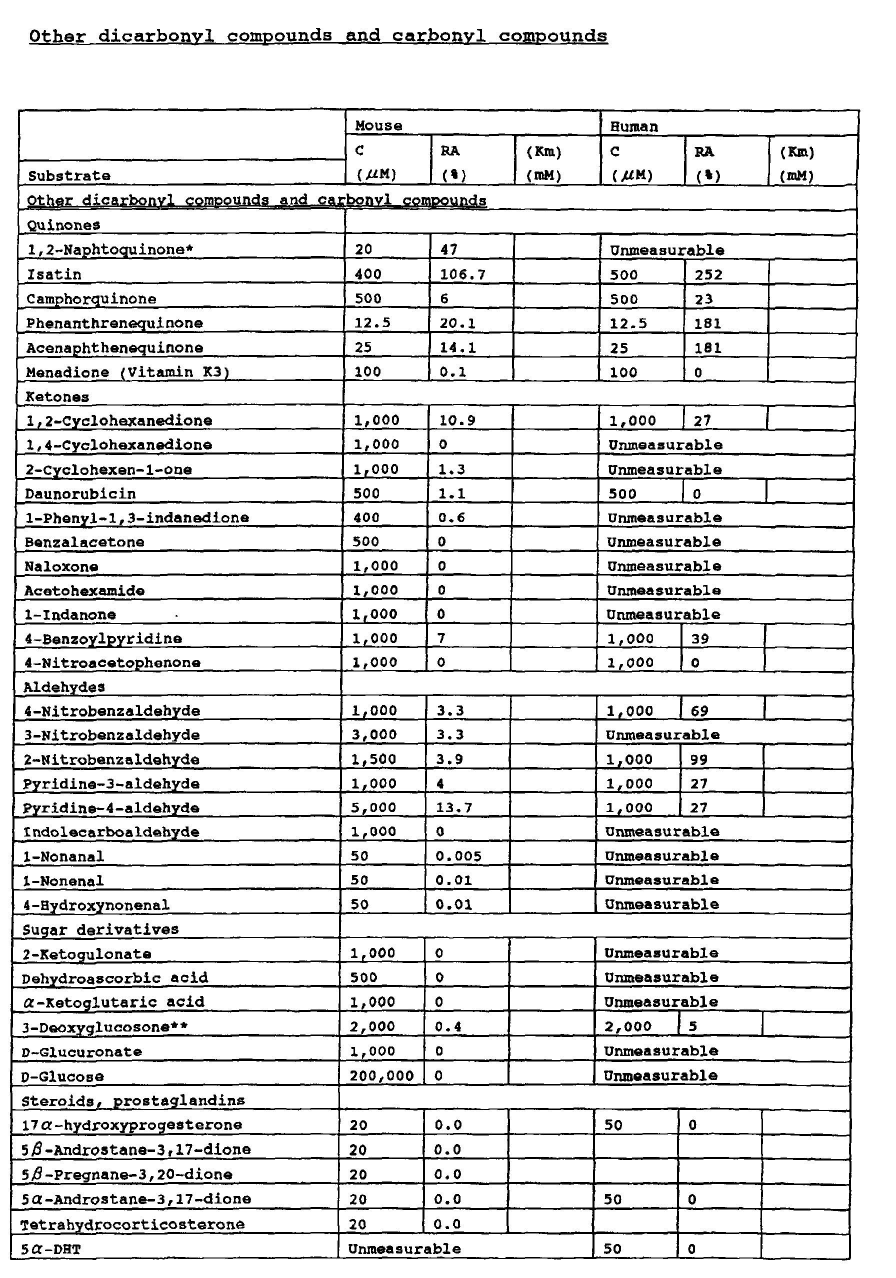

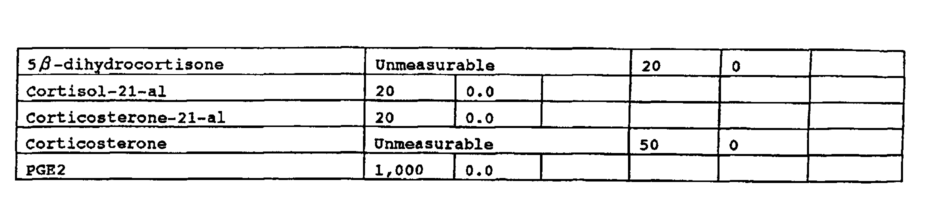

Table 2 below shows the rate constants for the major substrates for the mouse enzyme. 3,4-Hexanedione and 1-phenyl-1,2-propanedione were the best substrates, exhibiting the smallest Km values and the higher catalyst efficiency (Vmax/Km).

**Table 2**

| Substrate | Km (µM) | Vmax (unit/mg) | Vmax/Km (unit/mg/mM) |
|---|---|---|---|
| Pyruvic acid methyl ester | 1,700 | 2.7 | 1.6 |
| Benzoylformic acid methyl ester | 690 | 8.7 | 13 |
| Diacetyl(2,3-butanedione) | 900 | 6.1 | 6.7 |
| 1-Phenyl-1,2-propanedione | 140 | 6.7 | 48 |
| Acetoin | 6,500 | 0.51 | 0.08 |
| 2,3-Pentanedione | 280 | 5.7 | 20 |
| 2,3-Hexanedione | 230 | 4.1 | 18 |
| 3,4-Hexanedione | 130 | 6.6 | 51 |
| 2,3-Heptanedione | 200 | 4.4 | 22 |
| Pyridine-3-aldehyde | 7,900 | 0.62 | 0.08 |
| Pyridine-4-aldehyde | 4,400 | 0.78 | 0.18 |

Inhibitors for the enzyme reaction in the case of mouse DCR1 were evaluated and the results are shown in Table 3. Two enzymes were used here, mouse DCR1 and hamster liver diacetyl reductase (Hamster DR).

**Table 3**

| Compounds | Concentration (mM) | Inhibition (%) | |
|---|---|---|---|
| | | CDR1 | Hamster DR |
| Cibacron blue dye | 0.001 | 21 | 18 |
| Quercitin | 0.1 | 50 | 33 |
| Tetramethyleneglutaric acid | 0.1 | 0 | 63 |
| Ethacrynic acid | 0.1 | 0 | 3 |
| Benzoic acid | 0.1 | 32 | 59 |
| Anthranilic acid | 0.1 | 0 | 42 |
| Isonicotinic acid | 0.1 | 0 | 4 |
| Diphenlhydantoin | 0.1 | 17 | 2 |
| Barbital | 1 | 0 | 5 |
| Isobutylamine | 1 | 16 | 50 |
| Benzamide | 1 | 18 | 43 |
| 4-Hydroxybenzamide | 1 | 44 | 19 |
| o-Phenanthroline | 1 | 8 | 0 |
| Pyrazole | 10 | 29 | 5 |

The substrate reactivities obtained (Tables 1, 2 and 3) demonstrated that the dicarbonyl reductase of the invention has the following features.
(1) The enzyme of the invention is similar to mammalian (hamster) tissue diacetyl reductase in terms of its coenzyme specificity, pH dependency and substrate specificity. It is also similar to microorganic diacetyl reductase in terms of substrate specificity, but the microorganic enzyme is NADH-specific.
(2) It has strong reactivity for α-diketones with linear alkyl groups of 4-10 carbons, and especially diacetyl, 1,4-dibromo-2,3-butanedione, 1-phenyl-1,2-propanedione, 2,3-pentanedione, 2,3-hexanedione, 3,4-hexanedione and 2,3-heptanedione.
(3) It has strong reactivity for aromatic orthoquinones, and especially isatin, phenanthrenequinone and acenaphthenequinone.
(4) It has reactivity for pyruvic acid esters (methyl and ethyl), DL-glyceraldehyde and dimethyl-2-oxoglutarate.
(5) It also exhibits weak reactivity for numerous other substrates.
(6) Optimum pH: Based on their pH profiles, DCR1 and hDCR1 are believed to exhibit peak activity at below pH 6 (and pH 5). However, since one of their substrates, NADPH, is degraded at below pH 5, activity assay is not possible in that range when NADPH is used and the peak cannot be determined. The examples were therefore carried out at near pH 7, although the optimum pH is not limited to near 7.
(7) pH stability: For example, DCR1 and hDCR1 are incubated for 3 hours at 25°C in buffer solutions with different pH values, and the residual activity is then measured and compared and recorded as relative activity with respect to the value at pH 8.0 with no incubation.
(8) Effects of metal ions and inhibitors: After treating the enzyme with different metals and inhibitors, the residual activity was evaluated. MgCl₂ and CaCl₂ had virtually no effect on the enzyme. When mercury and cisplatin were given to the mice, expression of DCR1 increased.
(9) Molecular weight and subunits: Purified DCR1 and hDCR1 (with (His)-tag) both had a molecular weight of approximately 33 kDa according to sodium dodecylsulfate polyacrylamide gel electrophoresis (SDS-PAGE).

### (Application examples)

As explained in general terms above, the entirety or portions of the genes of the invention and proteins encoded thereby, or their mutated forms, may be used for diagnosis and treatment of diabetes complications as well as for various other applications, a few examples of which are explained in detail below.
(1) By detecting and quantifying the genes of the invention it is possible to estimate decomposition of dicarbonyl compounds in glycation.
(2) The proteins of the invention can directly degrade dicarbonyl compounds in glycation, and therefore pharmaceuticals containing these enzymes as components can suppress production of dicarbonyl compounds and thereby inhibit or suppress AGE production, to provide therapeutic agents for treatment of AGE-associated diabetes complications.
(3) The antisense oligonucleotides of the invention can control dicarbonyl reductase activity and therefore control decomposition of dicarbonyl compounds in glycation, to provide AGE production regulators. An AGE production regulator screening method is likewise provided.
(4) By detecting the genes of the invention and comparing their levels with normal expression levels it is possible to assay dicarbonyl reductase activity, to provide a method and diagnostic agent for diagnosis of AGE-associated diabetes complications based on the assay levels. There are likewise provided a method and diagnostic agent for diagnosis of AGE-associated diabetes complications, characterized by detection of protein DCR1.
(5) There are further provided a method and diagnostic agent for diagnosis of AGE-associated diabetes complications, characterized by using antibodies according to the invention.

The present invention will now be explained by way of examples which, however, are in no way intended to restrict the invention.

### Examples

### (Example 1) Cloning of gene dcr1

### (1) Determination of partial sequences of mouse renal glomeruli-specific genes

The renal glomerular fraction was purified from mice (C57BL/6) according to the method of Grant et al. (Eur. J. Biochem., 54, 531-540(1975)), and TRIzol (Gifco BRL) was used for extraction of the total RNA. The RNA was used as a template to construct a renal glomerular cDNA library according to the method of Okubo et al. (Nature Genet., 2, 173(1992)).

Next, 985 recombinants were randomly selected from the library, the recombinant DNA was extracted by a common method and the base sequences at the 3' end of the cDNA portion were determined. The sequences were determined using a DNA Sequencer (ABI PRISM 377) by PE Applied Biosystems and a reaction kit by the same manufacturer.

As a result of analyzing the expression frequency of each DNA fragment among the 985 recombinants, a gene expression frequency of 3/958 was found for the gene having Sequence-1 (238 residues).

### Sequence-1

GATCGTATCC CACTGGGCAA GTTCGNTGAG GTGGAGAACG TCGTGGACAC CATTCTCTTC 60
CTGCTGAGCA ACCGGAGTGG CATGACCACT GGCTCCACTT TGCCAGTGGA TGGGGGCTTC 120
CTGGCTACCT GAGCCCCCTG CCCACCGACA CTCTGCTCAG CTCTCTGCTC AGGACACTGT 180
GCCCTCCGCC CCTGCAATAA AGCTCTCTGC TCAGCCTGTG TGCTGATTCT CCAGGAAA 238

### (2) Obtaining DNA fragments containing Sequence-1

The DNA fragments containing Sequence-1 were obtained in the following manner.

### (2-1) Construction of cDNA library

The total RNA prepared in (1) of Example 1 was used as a template to synthesize cDNA using a CapFinder PCR cDNA Library Construction Kit (product of Clontech Laboratories).

### (2-2) Amplification of DNA fragments containing Sequence-1

First, an oligonucleotide (Sequence-2) comprising part of Sequence-1 was synthesized with a DNA synthesizer (ABI 380B) by PE Applied Biosystems.
Sequence-2 5' CAGTGTCCTGAGCAGAGAGCTGAG 3'

The cDNA library constructed in (2-1) was then used as a template for PCR with the oligonucleotide of Sequence-2 and the oligonucleotide supplied with the kit as primers. A kit by Takara Shuzo, KK. (Takara LA PCR Kit Ver.2) and PCR Thermal Cycler Mp by Takara Shuzo, KK. were used for the reaction.

### Reaction composition:

| | |
|---|---|
| cDNA library | 5 µl |
| 10xPCR buffer (containing 25 mM Mg²⁺) | 5 µl |
| 2.5 mM dNTP | 1 µl |
| 10 µM oligonucleotide (Sequence-2) | 2 µl |
| Kit-supplied oligonucleotide | 2 µl |
| Water | 34.5 µl |
| LA Tag polymerase | 0.5 µl |
| Total | 50 µl |

### Reaction conditions:

After reaction at 94°C for 30 seconds, cooling to 60°C at a rate of -1°C/2 seconds and maintaining a temperature of 60°C for 30 seconds, the mixture was heated to 72°C and held at that temperature for 4 minutes. This procedure was repeated 30 times to amplify the target sequence.

This procedure accomplished specific amplification of a DNA fragment (approximately 0.8 kb) having a portion of Sequence-1.

### (3) Subcloning in base sequence determining vector

The DNA fragment amplified in (2) was fractionated by agarose gel electrophoresis (gel concentration: 1%) according to a common method. After staining the gel with ethidium bromide, it was exposed to ultraviolet light and the gel containing the target bands was cut out. The DNA fragments were extracted and purified from the agarose gel using a GENECLEAN II Kit (product of Bio101).

The purified DNA fragments were subcloned by the method described below in the base sequence determining vector, pT7Blue T-Vector (product of Novagene). The ligation solution was reacted therewith at 16°C for 16 hours using a kit (Takara DNA Ligation Kit Ver.2) by Takara Shuzo, KK.

### Reaction composition:

| | |
|---|---|
| PCR product | 1 µl (50 ng) |
| T7 Blue T-Vector | 1 µl |
| Water | 3 µl |
| Ligation solution | 5 µl |
| Total | 10 µl |

This reaction solution was used for transformation of *E. coli* K12 DH5 by a common method. The transformants were plated on LB agar medium containing 50 µg/ml ampicillin (Amp), 40 µg/ml 5-bromo-4-chloro-3-indolyl-β-d-galactoside (β-gal) and 100 µM of isopropyl-β-d-thio-galactopyranoside (IPTG), and were cultured overnight at 37°C. The white colonies were seeded in 10 ml of LB liquid medium containing 50 µg/ml Amp and cultured overnight at 37°C, and after collecting the cells by centrifugation, the recombinant DNA was purified with a QIAprep Spin Plasmid Miniprep Kit (product of Qiagen).

### (4) Base sequence determination of DNA fragments

A DNA Sequencer by PE Applied Biosystems was used for the base sequence determination, by the dye terminator method. The determined base sequences were used for the basis of oligonucleotide synthesis, and the total base sequence was determined by the primer walking method (sequence No.1 of the Sequence Listing). The base sequences of both strands were determined, and the base sequences of 2 independent clones were found to be completely identical. This base sequence included the Sequence-2 and a region of Sequence-1 upstream of Sequence-2, thus confirming that the target gene (gene dcr1) had been cloned. The full base sequence including gene dcr1 is shown below.

TTGCTGCGAG AAGACGACAG AATGGGAGGA CCAGGCTCAA GCACCATGGA 50
CCTGGGGCTT GCAGGTCGGC GGGCGCTGGT CACCGGCGCG GGCAAAGGCA 100
TCGGGCGCAG CACTGTGCTG GCGTTGAAGG CGGCTGGTGC ACAGGTTGTG 150
GCGGTGAGTC GGACGCGAGA GGACCTGGAC GACCTGGTCC GCGAGTGTCC 200
TGGTGTGGAG CCTGTGTGTG TGGACCTGGC CGACTGGGAG GCCACAGAGC 250
AGGCCCTAAG CAATGTGGGA CCCGTGGACC TTCTGGTGAA CAATGCTGCT 300
GTGGCTCTGT TGCAGCCCTT CCTGGAGGTC ACCAAGGAGG CCTGTGACAC 350
ATCCTTCAAT GTGAATCTTC GGGCTGTCAT CCAGGTGTCT CAGATTGTGG 400
CCAAGGGCAT GATAGCTCGG GGAGTTCCAG GAGCCATTGT GAATGTCTCC 450
AGCCAGGCCT CCCAACGTGC ACTGACCAAC CATACTGTCT ACTGTTCCAC 500
CAAGGGTGCT CTGGACATGT TGACCAAGAT GATGGCCCTA GAGCTTGGGC 550
CCCACAAGAT CCGTGTGAAT GCAGTAAACC CCACAGTAGT GATGACACCC 600
ATGGGCCGGA CCAACTGGAG TGACCCCCAC AAAGCTAAGG CCATGCTGGA 650
TCGTATCCCA CTGGGCAAGT TCGCTGAGGT GGAGAACGTC GTGGACACCA 700
TTCTCTTCCT GCTGAGCAAC CGGAGTGGCA TGACCACTGG CTCCACTTTG 750
CCAGTGGATG GGGGCTTCCT GGCTACCTGA GCCCCCTGCC CACCGACACT 800
CTGCTCAGCT CTCTGCTCAG GACACTGTGC CCTCCGCCCC TGCAATAAAG 850
CTCTCTGCTC AGCCTGTGTG CTGATTCTCC AGGAAA 886

### (Example 2) Production of protein DCR1

### (1) Construction of expression vector pHis-glB

The cDNA fragment containing gene dcr1 obtained in Example 1 was amplified by PCR using the primers shown below. The PCR conditions were the same as described in Example 1.
Sequence-3 5' TAATGGATCCATGGACCTGGGGCTTGCAGGTCGG 3'
Sequence-4 5' ATTAGAATTCAGGTAGCCAGGAAGCCCCCATCC 3'

The amplified and purified cDNA fragment was cleaved with restriction enzymes EcoRI and BamHI (both by Takara Shuzo). After cleavage, fractionation was again performed by agarose gel electrophoresis to purify an approximately 0.7 kb DNA fragment (Fragment-1).

The protein production vector pTrcHisA (product of Invitrogen) was cut with restriction enzymes EcoRI and BamHI in the same manner as above and the opened vector was purified.

Fragment-1 and the opened vector were combined and used for ligation and transformation of *E. coli* K12 under the conditions described in Example 1, and after culturing the transformants and collecting the cells by centrifugation, the recombinant DNA was purified therefrom. The recombinant DNA constructed in this manner was designated as pHis-glB.

The expression conditions and purification procedure are described below.

The medium (L(+)-glucose) contained the following components (in 1 liter)

| | |
|---|---|
| Tripticase peptone | 10 g |
| NaCl | 5 gr |
| Yeast extract | 5 gr |
| D-glucose | 2 gr |

Pre-culturing was carried out by shake culturing overnight at 37°C using the same medium as above (8 ml) containing 200 µg/ml ampicillin.

The main culturing was carried out at 37°C using the same medium as above (150 ml) containing 200 µg/ml ampicillin, and at the point where OD=0.6, 100 mg of IPTG (approximately 3 mM) was added for an additional 3 hours of culturing.

After cooling on ice, the cells were collected, suspended in the following column buffer and lyophilized at -20°C.
- Column buffer:: 10 mM Tris-HCl, pH 8.0
200 mM NaCl
0.1 mM PMSF

After liquefaction, 20 mg of lysozyme, 5 µl each of DNaseI (5 mg/ml) and RNaseA (5 mg/ml) and 100 µl of 10% TritonX-100 were added for lysis at 37°C for 15 minutes.

The supernatant was drawn after centrifugation at 9000 rpm, 4°C, 10 minutes, 10 ml of fresh column buffer was added to the supernatant, and then 1 ml bed volume of Nickel-NTA-agarose gel (Qiagen) was added prior to gentle shaking at room temperature for 15 minutes.

This was packed into a glass column for purification under the following conditions.
Washing column buffer (50 ml):
   column buffer + 50 ml of 10 mM imidazole
   column buffer + 10 ml of 30 mM imidazole
Elution column buffer + 500 mM imidazole
Dialysis column buffer (4°C)

After adding 15 vol/vol% glycerol, the mixture was stored at -20°C.

The purification check was made by the Coumassie blue staining method after SDS-PAGE.

The protein amount was measured by the HS Bradford method (Biorad).

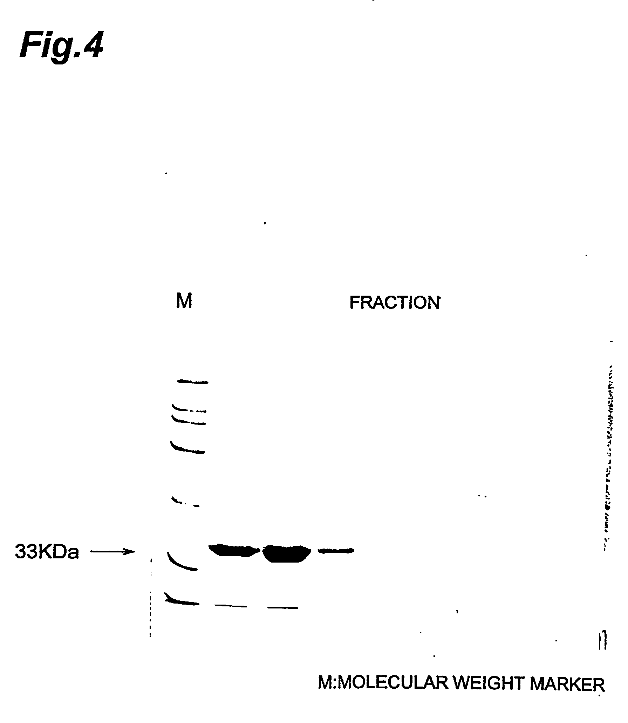

Fig. 4 shows affinity purification of the recombinant protein DCR 1.

The molecular weight was approximately 33 kDa, and the purity was estimated to be at least 95% (yield: approx. 3 mg (150 ml *E. coli* medium)).

### (Example 3) Antibody preparation

Anti-DCR protein antibodies were prepared according to the method of Sawaday Biotechnology, using the following antigens.

Ac-NH-SRTREDLDDLVREC-COOH was used as the antigen for preparation of antibody 731 and Ac-NH-QASQRALTNHTVYC-COOH as the antigen for preparation of antibody 733, and rabbits were immunized with them in conjugate form.

Specifically, the antigen was used as a carrier protein and hemocyanin (KLH) was bound thereto, for immunization of rabbits 6 times in a period of 2 months at 150 µg per administration, to obtain anti-peptide antibodies.

After subjecting 0.1 µg of the recombinant obtained in Example 2 to SDS-PAGE, it was transferred from the gel to a Hybond-ECL nitrocellulose membrane (Amersham) using a Miniprotein II blotting apparatus (Biorad).

The protein-blotted membrane filter was washed with distilled water and then each of the prepared antibodies was used for detection with an ECL Western Blotting Detection System by Amersham. As a result, detection was possible as a band with an apparent molecular weight of approximately 33 kDa. This antibody can therefore be used for antibody assay using different cells.

### (Example 4) Expression of protein DCR1

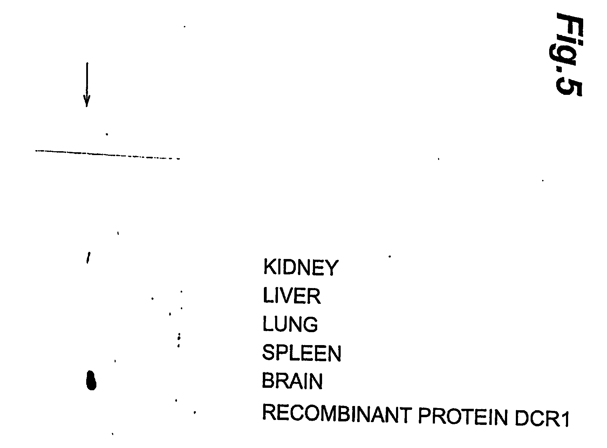

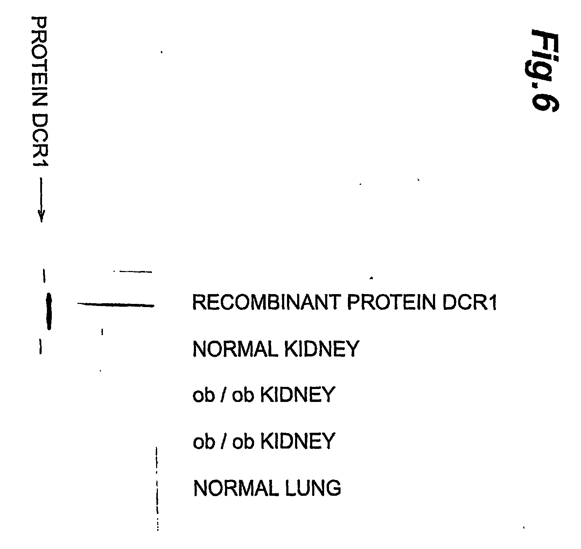

(1) Fig. 3 shows the expression of protein DCR1 in the primary organs of normal mice, according to the Western blot method. The antibodies used were antibodies #731 and #733 prepared in Example 3. Each organ extract contained 100 µg of protein. A 0.1 µg portion of recombinant protein was used.
   The organ extracts were prepared by adding 4 ml of buffer solution (Tris-HCl buffer solution containing 0.2% TritonX-100, 0.1 mM PMSF, 0.15 M NaCl, 1 mM MgCl₂ and 0.5 mM EDTA/EGTA, pH 8.0) to kidney tissue from 5 mice, and subjecting it 4 or 5 times to ultrasonic treatment for 30 seconds with a SONIC BLENDER (HITACHI HG30).
   The supernatant obtained by centrifugation at 100,000 x g for 1.5 hours was then fractionated and the precipitate obtained by an additional 30 minutes of centrifugation was lyophilized at -80°C and provided for various experiments.
   Both antibodies show specific expression of the protein in the kidneys. Antibody #733 reacts with protein DCR1 with greater specificity, exhibiting lower cross-reaction with isozymes. The results shown in Figs. 5 and 6 are those obtained using antibody #733.
(2) Fig. 6 shows the results of Western blot measurement of the expression of protein DCR1 in kidney extracts from pathological model mice, in comparison to healthy mice.

The antibodies used were antibody #731 and the specific antibody #733. The protein amount in each organ extract was 150 µg, and a 0.1 µg portion of recombinant protein DCR1 was used.

The following table shows pathological severity in terms of blood sugar levels, in correlation with the reduction trend for protein DCR1.

| Average for 5 mice | ob/ob | Control | db/db | Control |
|---|---|---|---|---|
| Body weight (g) | 45.6 | 20.3 | 42.5 | 20.6 |
| Blood sugar level (mg/dl) | 335 | 195 | 512 | 168 |

These results shows a reduction trend for protein DCR1 that is conversely related to pathological severity (blood sugar level).

### (Example 5) Enzyme activity

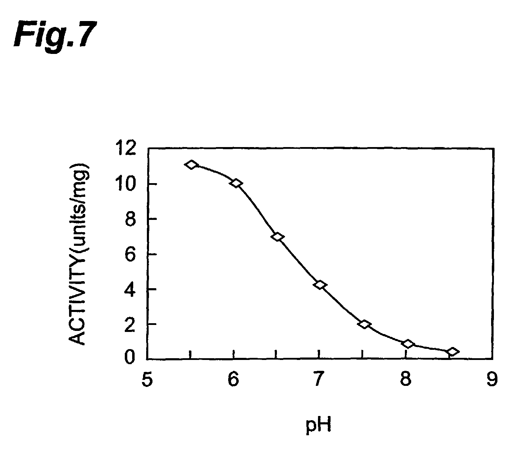

(1) This was assayed under the following conditions.
   The reaction mixture (total: 2.0 ml) comprised an 80 mM potassium phosphate solution (pH 7.0 or 6.0), 0.1 mM NADPH or NADH and a carbonyl substrate or enzyme (50 µl) (10.1 µg or 3.7 µg of glomerin B).
   The oxidation rate for NAD(P)H was measured at 340 nm.
   One unit of enzyme activity was defined as that which catalyzed oxidation of 1 µmol of NAD(P)H in one minute at 25°C.
(2) The substrate specificity of protein DCR1 for carbonyl compounds is summarized in Table 1.
(3) pH dependency of diacetyl reductase activity due to protein DCR1
   The activity was measured using 10 mM diacetyl, and the specific activity was calculated based on the protein concentration in the protein DCR1 sample (Fig. 7).

The compounds for which no activity was found were 2-cyclohexen-1-ol, S-indan-1-ol, 4-nitroacetophenone and menadione (vitamin K3).

### (Example 6) Cloning of human-type homologue

(1) Construction of cDNA
A cDNA library was constructed from human kidney mRNA.
The human kidney mRNA used was Human Kidney PolyA⁺ RNA found in CATALOG #6538-1 by Clontech (Chomczynski, et al., Anal. Biochem. 162:156-159(1987), Sambrook, J., et al., (1989) Molecular Cloning: A Laboratory Manual, 2nd ed. (Cold Spring Harbor Laboratory, Cold Spring Harbor, NY), pp.6.22-6.34)).
The library was constructed using a SMART™ PCR Library Construction Kit by Clontech, according to the manual supplied with the kit. The necessary primers, reagents, etc. used were those supplied with the kit.
Single-stranded DNA was prepared according to the same kit. After the reaction, the mRNA used as the template was digested and removed with RnaseH (product of Gifco). Agarose gel electrophoresis was then performed and the low molecular weight impurities were removed for purification using a Sepaglass band prep kit by Pharmacia.
The obtained single-stranded DNA was finally prepared in a 25 µl solution.
(2) Construction of double-stranded cDNA fragment
A SMART™ PCR Library Construction Kit was used for LD-PCR to construct a cap site-containing double-stranded cDNA fragment, according to the manual supplied with the kit. The EST data obtained above from mice were used to design the following 3' end primer having a sequence downstream from the termination codon.
Primer #1162: 5'-ccaagcttgtaggatgagcacggcatgg-3' (28mer)

The LD-PCR was carried out under the following conditions using a Takara Thermal Cycler MP by Takara Shuzo.

| | | |
|---|---|---|
| 1 cycle | 95 | 1 min |
| 20 cycles | 95 | 30 sec |
| | 55 | 2 min |
| | 72 | 3 min |
| 1 cycle | 95 | 1 min |
| | 55 | 3 min |
| | 72 | 10 min |

After completion of the reaction, the protein was removed with phenol:chloroform:isoamyl alcohol (25:24:1), and the DNA was recovered by ethanol precipitation. This was further subjected to agarose gel electrophoresis (gel concentration: 1.0%), and after staining the gel with ethidium bromide it was exposed to ultraviolet light and the gel portions containing the target bands were cut out. The extraction and purification of the DNA fragments from the agarose gel was accomplished using a Sepaglass™ Band PrepKit by Pharmacia.
(3) The cDNA fragment obtained in (2) above was again amplified by PCR using a nested PCR primer only at the 3' end. The 3' end primer used had the following sequence.
Primer #1163: 5'-ccaagcttgaggtgtgtggagggagc-3'

The PCR was carried out under the following conditions using a Takara Thermal Cycler MP by Takara Shuzo.

| | | |
|---|---|---|
| 1 cycle | 95 | 1 min |
| 20 cycles | 95 | 30 sec |
| | 55 | 2 min |
| | 72 | 3 min |
| 1 cycle | 95 | 1 min |
| | 55 | 3 min |
| | 72 | 10 min |

After completion of the reaction, the protein was removed with phenol:chloroform:isoamyl alcohol (25:24:1), and the DNA was recovered by ethanol precipitation. This was further subjected to agarose gel electrophoresis (gel concentration: 1.0%), and after staining the gel with ethidium bromide it was exposed to ultraviolet light and the gel portions containing the target bands were cut out. The extraction and purification of the DNA fragments from the agarose gel was accomplished using a Sepaglass™ Band PrepKit by Pharmacia.
(4) The cDNA fragment obtained in (3) above was subcloned in plasmid DNA in the following manner. The protocol followed was that of the pGEM^{R}-T Easy Vector Systems manual by Promega (Promega, Printed in USA. Revised 7/79; Part #TM042, TECHNICAL MANUAL pGEM-T and pGEM-T Easy Vector Systems, INSTRUCTIONS FOR USE OF PRODUCTS A160, A1380, A3600 AND A3610), and the supplied materials were used.

The ligation solution and conditions were as follows, with reaction at 40°C for 3 days (60 hours).

### Reaction composition:

| | |
|---|---|
| PCR product | 2 µl (140 ng) |
| T4 DNA Ligase 10 x Buffer | 1 µl |
| pGEM^{R}-T Easy Vector | 1 µl (50 ng) |
| T4 DNA Ligase | 1 µl |
| Water | 5 µl |
| Total | 10 µl |

This reaction solution was used for transformation of strain JM109 by a common method. The transformants were cultured overnight at 37°C with 50 µg/ml of ampicillin (Amp). Fifty colonies were prepared on a separate master plate, transferred to a membrane (Hybond+, product of Amersham) and hybridized with a DIG-labeled 3' oligonucleotide, and the positive clones were selected, combined into a single colony and purified. This was then seeded in 10 ml of LB liquid medium containing 50 µg/ml Amp and cultured overnight at 37°C, and after collecting the cells by centrifugation the recombinant DNA was purified.
(5) Determining base sequence of DNA fragment
   The base sequence was determined by the dye terminator method using a DNA Sequencer by PE Applied Biosystems. The fragment lengthb was as short as about 750 bp thus allowing the use of the general use primers SP6 and T7, and thus the base sequence was determined without primer walking.
(6) Based on the sequence data obtained in (5) there was designed a new 5' primer from a region further upstream from the site corresponding to Met1 at the 5' end of the open reading frame (ORF).
   Primer #1446: 5'-ggaattcgattccaagcttgt-3' (21mer)

   This primer was combined with the 3' primer #1162 comprising the sequence of the portion downstream from the termination codon at the 3' end of the ORF, which had already been designed based on the EST data, and a PCR was carried out with a low number of cycles using as the template the original single-stranded cDNA obtained in (1) above, to obtain a standard clone. Here, both since the primers at the 5' and 3' ends were both unique, a clone was obtained with no misreaction by one PCR of 20 cycles.

### Reaction conditions:

| | |
|---|---|
| cDNA | 2.5 µl |
| Water | 36.5 µl |
| 10 x PCR buffer | 5 µl (Perkin Elmer) |
| 2 mM dNTP mix | 5 µl (Perkin Elmer) |
| 5' primer: #1446 | 1 µl |
| 3' primer: #1162 | 1 µl |
| AmpliTaq Polymerase | 0.5 µl (Perkin Elmer) |

The PCR was carried out under the following conditions using a Takara Thermal Cycler MP by Takara Shuzo.

| | | |
|---|---|---|
| 1 cycle | 95 | 1 min |
| 20 cycles | 95 | 30 sec |
| | 55 | 2 min |
| | 72 | 3 min |
| 1 cycle | 95 | 1 min |
| | 55 | 3 min |
| | 72 | 10 min |

After completion of the reaction, the protein was removed with phenol:chloroform:isoamyl alcohol (25:24:1), and the DNA was recovered by ethanol precipitation. This was further subjected to agarose gel electrophoresis (gel concentration: 1%), and after staining the gel with ethidium bromide it was exposed to ultraviolet light and the gel portions containing the target bands were cut out. The extraction and purification of the DNA from the agarose gel was accomplished using a Sepaglass™ Band PrepKit by Pharmacia.

The purified cDNA was subcloned in plasmid DNA by the same procedure used in (4) and (3) above (pGEM^{R}-T Easy Vector Systems manual by Promega (Promega, Printed in USA. Revised 7/79; Part #TM042, TECHNICAL MANUAL pGEM-T and pGEM-T Easy Vector Systems, INSTRUCTIONS FOR USE OF PRODUCTS A160, A1380, A3600 AND A3610)).

The ligation solution and conditions were as follows, with reaction at 40°C for 60 hours.

### Reaction composition:

| | |
|---|---|
| PCR product | 3 µl (150 ng) |
| T4 DNA Ligase 10 x Buffer | 1 µl |
| pGEM^{R}-T Easy Vector | 1 µl (50 ng) |
| T4 DNA Ligase | 1 ul |
| Water | 4 µl |
| Total | 10 µl |

This reaction solution was used for transformation of strain JM109 by a common method. The transformants were plated on LB agar medium containing 50 µg/ml ampicillin (Amp) and cultured overnight at 37°C. Twenty of the white colonies were transferred to a separate master plate, transferred to a membrane and hybridized with a DIG-labeled 3' oligonucleotide, and the positive clones were selected and isolated as a single colony. This was then seeded in 10 ml of LB liquid medium containing 50 µg/ml Amp and cultured overnight at 37°C, and after collecting the cells by centrifugation the recombinant DNA was purified.

The base sequences for a number of clones were determined. The sequence primers used were #1446 and #1162 mentioned above. The apparatus used was a DNA Sequencer by PE Applied Biosystems, according to the dye terminator method. The ORF portion was completely included within the primers. The determined base sequence is listed as sequence No.3 in the Sequence Listing.

### (Example 7) Production of protein hDCR1

### (1) Construction of expression vector pHis-glB

The cDNA fragment containing protein hdcr1 obtained in Example 6 was amplified by the PCR using the primers shown below. The PCR was carried out under the same conditions described in Example 6.
Sequence #1448: caggatccatggsgctgttcctcgcgggc
Sequence #1449: cagaattctcagcaggcccagaagcccccttc

The amplified and purified cDNA fragment was cut with restriction enzymes EcoRI and BamHI (both by Takara Shuzo). The cutting treatment was followed by further fractionation by agarose gel electrophoresis to purify an approximately 0.75 kb DNA fragment (Fragment-1).

The protein production vector pTrcHisA (product of Invitrogen) was cleaved with restriction enzymes EcoRI and BamHI in the same manner as above and the opened vector was purified.

Fragment-1 and the opened vector were combined and ligated and then used for transformation of *E. coli* K12 under the conditions described in Example 6, and after culturing the transformants and collecting the cells by centrifugation, the recombinant DNA was purified therefrom. The recombinant DNA constructed in this manner was designated as pTrcHisAhuglB.

The expression conditions and purification procedure are described below.

The medium (L(+)-glucose) contained the following components (in 1 liter)

| | |
|---|---|
| Tripticase peptone | 10 g |
| NaCl | 5 gr |
| Yeast extract | 5 gr |
| D-glucose | 2 gr |

Pre-culturing was carried out by shake culturing overnight at 37°C using the same medium as above (8 ml) containing 200 µg/ml ampicillin.

The main culturing was carried out at 37°C using the same medium as above (150 ml) containing 200 µg/ml ampicillin, and at the point where OD=0.6, 100 mg of IPTG (approximately 3 mM) was added for an additional 3 hours of culturing.

After cooling on ice, the cells were collected, suspended in the following column buffer and lyophilized at -20°C.
- Column buffer:: 10 mM Tris-HCl, pH 8.0
200 mM NaCl
0.1 mM PMSF

After liquefaction, 20 mg of lysozyme, 5 µl each of DNaseI (5 mg/ml) and RNaseA (5 mg/ml) and 100 µl of 10% TritonX-100 were added for lysis at 37°C for 15 minutes.

The supernatant was drawn after centrifugation at 9000 rpm, 4°C, 10 minutes, 10 ml of fresh column buffer was added to the supernatant, and then a 1 ml bed volume of Nickel-NTA-agarose gel (Qiagen) was added prior to gentle shaking at room temperature for 15 minutes.

This was packed into a glass column for purification under the following conditions.
Washing column buffer (50 ml):
   column buffer + 50 ml of 10 mM imidazole
   column buffer + 10 ml of 30 mM imidazole
Elution column buffer + 500 mM imidazole
Dialysis column buffer (4°C)

After adding 15 vol/vol% glycerol, the mixture was stored at -20°C.

The purification check was made by the Coumassie blue staining method after SDS-PAGE.

The protein amount was measured by the HS Bradford method (Biorad). The molecular weight was approximately 33 kDa, and the purity was estimated to be at least 95% (yield: approx. 3 mg (150 ml *E. coli* medium)).

### (Example 8) Enzyme activity

The reaction mixture (total: 2.0 ml) comprised an 80 mM potassium phosphate solution (pH 7.0 or 6.0), 0.1 mM NADPH or NADH and a carbonyl substrate or enzyme (50 µl) (10.1 µg or 3.7 µg of hDCR1).

The oxidation rate for NAD(P)H was measured at 340 nm.

One unit of enzyme activity was defined as that which catalyzed oxidation of 1 µmol of NAD(P)H in one minute at 25°C.

The measured values were determined by subtracting the value for a reaction system of each substrate containing no enzyme, and were expressed as relative activity with respect to the diacetyl reduction activity.
(2) The results of measuring the substrate specificity of protein hDCR1 for different carbonyl compounds is summarized in Table 1.

### Industrial Applicability

Dicarbonyl compounds, typical of which is diacetyl, are intermediate metabolites in the pathway of production of diabetes complication-implicated AGE from reducing sugars and proteins in the body, and they are very highly reactive compounds. The dicarbonyl reductases of the invention are enzymes that suppress production of AGE by degrading these dicarbonyl compounds that are intermediate metabolites, and they are understood to play an important role in the onset and progression of diabetes complications. The present invention is therefore believed to be useful for the development of therapeutic agents designed to suppress onset of diabetes complications, sugar metabolism disorders in kidney failure, AGE production and the arteriosclerosis that accompanies it, while the recombinant proteins themselves can be used for detoxification of dicarbonyl compounds that are the AGE production precursors in the blood.

By using the genes of the invention it is possible to provide expression regulating factors for the genes, by way of a screening method for expression regulating factors for the genes.

By using the proteins of the invention it is possible to provide activity regulators for the proteins, by way of a screening method for activity regulators for the proteins.

These can also be used as prophylactic and therapeutic agents for kidney failure and other conditions known as diabetes complications, or as materials for the creation of such agents.

## Claims

1. A dicarbonyl reductase having high reducing activity for α-diketones with linear alkyl groups of 4-10 carbons and for aromatic orthoquinones.

2. A dicarbonyl reductase according to claim 1, characterized in that the α-diketones are one or more diketones selected from the group consisting of diacetyl, 2,3-pentanedione, 2,3-hexanedione, 3,4-hexanedione and 2,3-heptanedione, and the aromatic orthoquinones are one or more quinones selected from the group consisting of isatin, phenanthrenequinone and acenaphthenequinone.

3. A gene coding for a protein as described in (a) or (b) below.
(a) A protein comprising the amino acid sequence set forth in SEQ ID NO: 2 or NO: 4 in the Sequence Listing.
(b) A protein comprising an amino acid sequence which is the amino acid sequence set forth in SEQ ID NO: 2 or NO: 4 in the Sequence Listing with a deletion, substitution or addition of one or more amino acids, and having dicarbonyl reductase activity.

4. A gene coding that hybridizes with a gene according to claim 3 under stringent conditions and codes for a protein with dicarbonyl reductase activity.

5. A protein as described in (a) or (b) below.
(a) A protein comprising the amino acid sequence set forth in SEQ ID NO: 2 or NO: 4 in the Sequence Listing.
(b) A protein comprising an amino acid sequence which is the amino acid sequence set forth in SEQ ID NO: 2 or NO: 4 in the Sequence Listing with a deletion, substitution or addition of one or more amino acids, and having dicarbonyl reductase activity.

6. An antibody for a protein according to claim 5.

7. An antisense oligonucleotide having the full or partial sequence of the antisense chain for a gene according to claim 3 or 4, which inhibits biosynthesis of a protein according to claim 5.

8. A dicarbonyl compound production inhibitor characterized by containing a protein according to claim 5.

9. An AGE production inhibitor characterized by containing a protein according to claim 5.

10. An AGE production regulator that contains an antisense oligonucleotide according to claim 7.

11. A gene expression regulating factor screening method that employs at least a portion of a gene according to claim 3.

12. A gene expression regulating factor obtained by the method of claim 11.

13. A protein activity regulator screening method that employs at least a portion of a protein according to claim 5.

14. A protein activity regulator obtained by the method of claim 13.
